# EUROPEAN PATENT APPLICATION

(11) **EP 4 512 344 A1**
(43) Date of publication of application: **26.02.2025**
(21) Application number: 23198158.0
(22) Date of filing: 19.09.2023
(51) Int. Cl.: A61B 8/08, A61B 8/00

(54) **MOTION PREDICTION FOR ZOOM STABILIZATION SYSTEMS AND METHODS**

(30) Priority: 22.08.2023 US 202363533933 P
(71) Applicant: Koninklijke Philips N.V., 5656 AG Eindhoven (NL)
(72) Inventor: LONG, Willie Jie, Eindhoven (NL); JIANG, Wei, Eindhoven (NL); CLARK, David Wesley, 5656 AG Eindhoven (NL); ADAMS, Darwin Philip, Eindhoven (NL)
(74) Representative: Philips Intellectual Property & Standards

(57) **Abstract**

Techniques for modifying a zoom region of interest in medical images are disclosed. A zoom region of interest is identified in an image, and the zoom region of interest is displayed. A target object is identified in the zoom region of interest. A set of images each including the target object is stored in a first-in first-out buffer. Movement of the target object in the set of images is determined, and a location of the target object in a subsequent image is predicted based on the movement of the target object. Based on the predicted location of the target object in the subsequent image, the zoom region of interest is modified, and the subsequent image is displayed using the modified zoom region of interest.

## Description

### FIELD OF THE INVENTION

This application relates to predicting motion in medical images. More specifically, this application relates to predicting motion, such as deformation and/or displacement, for stabilized zooming in medical images.

### BACKGROUND OF THE INVENTION

Various medical imaging modalities can be used for clinical analysis and medical intervention, as well as visual representation of the function of organs and tissues, such as magnetic resonance imaging (MRI), ultrasound (US), or computed tomography (CT). Implementations of medical imaging modalities may provide a zoom feature, which can be used to isolate and magnify specific targets within a region of interest (ROI), such as by cropping the full image and/or reacquiring a sub-image. The ROI is typically user-selected, static (e.g., fixed size and location), and non-adaptive (i.e. independent of image or other acquired data).

### SUMMARY OF THE INVENTION

Apparatuses, systems, and methods for stabilized zooming based on motion prediction are disclosed. As used herein, motion can refer to movement of one or more objects within a ROI and/or movement of an imaging device, such as a US probe. For example, the disclosed systems and methods can be used to stabilize a ROI when the US probe is purposefully or accidentally moved and/or when the one or more objects deform or otherwise move during imaging, such as due to biological cycles (e.g., breathing or a heartbeat) or patient movement.

In accordance with at least one example disclosed herein, an ultrasound imaging system is disclosed. The ultrasound imaging system comprises an ultrasound probe configured to acquire ultrasound image data and at least one processor in communication with the ultrasound probe. The at least one processor is configured to identify a zoom region of interest in an image of the ultrasound image data and cause display of the zoom region of interest in the image. The at least one processor is further configured to identify a target object in the zoom region of interest, and to cause a set of images of the ultrasound image data to be stored in a first-in first-out buffer, wherein each image in the set of images includes the target object. The at least one processor is configured to determine movement of the target object in the set of images and predict a location of the target object in a subsequent image based on the determined movement of the target object in the set of images. Furthermore, the at least one processor is configured to modify the zoom region of interest based on the predicted location of the target object in the subsequent image and cause display of the modified zoom region of interest in the subsequent image.

In some implementations, the at least one processor is configured to cause the set of images of the ultrasound image data to be stored adaptively in the first-in first-out buffer.

In accordance with at least one example disclosed herein, a method, preferably a computer-implemented method, of modifying a zoom region of interest in medical images is disclosed. The method comprises identifying a zoom region of interest in an image of medical imaging data. The method further comprises causing display of the zoom region of interest in the image, and identifying a target object in the zoom region of interest. The method further comprises storing, preferably adaptively, a set of images of the medical imaging data in a first-in first-out buffer, wherein each image in the set of images includes the target object. The method further comprises determining movement of the target object in the set of images, and predicting a location of the target object in a subsequent image based on the determined movement of the target object in the set of images. The method further comprises modifying the zoom region of interest based on the predicted location of the target object in the subsequent image, and causing display of the modified zoom region of interest in the subsequent image.

In accordance with at least one example disclosed herein, a non-transitory computer-readable medium is disclosed carrying instructions that, when executed, cause a processor to perform operations to perform at least one method disclosed herein.

In some implementations, the zoom region of interest is modified based on the predicted location of the target object in the subsequent image such that the target object is displayed on a display in a same location in the image and the subsequent image.

In some implementations, the movement of the target object corresponds to at least one of displacement of the target object, deformation of the target object, displacement of tissue, deformation of tissue, or motion of the ultrasound probe between images of the set of images.

In some implementations, the target object and/or the zoom region of interest is identified automatically.

In some implementations, the target object is identified based on at least one of backscatter intensity, signal-to-noise ratio, flow velocity, or flow power.

In some implementations, the target object comprises a target anatomy or a medical treatment device.

In some implementations, storing the set of images includes changing membership in the set of images.

In some implementations, the storing of the set of images, the determining the movement of the target object, the predicting of the location of the target object in the subsequent image, and the modifying of the zoom region of interest are performed iteratively. In some implementations, the iterative performing is terminated in response to detecting an exit criterion. In some implementations, the exit criterion comprises movement of the target object beyond a threshold, receiving a user input, or receiving a system input. In some implementations, detecting the exit criterion includes calculating a confidence metric based on at least one tracking variable, wherein the tracking variable includes at least one of a correlation coefficient or a mean squared error.

In some implementations, non-uniform transmit sequencing and/or non-uniform receive beamforming is performed to acquire the ultrasound image data at a first resolution within the zoom region of interest and at a second resolution outside of the zoom region of interest, wherein the first resolution is higher than the second resolution.

In some implementations, the zoom region of interest is identified automatically.

In some implementations, displaying the zoom region of interest comprises magnifying the image or acquiring a different image.

In some implementations, displaying the modified zoom region of interest in the subsequent image comprises presenting at least a portion of a previously-acquired image.

In some implementations, modifying the zoom region of interest based on the predicted location of the target object in the subsequent image comprises determining a degree of zooming in the subsequent image.

### BRIEF DESCRIPTION OF THE DRAWINGS

Fig. 1 is a block diagram of an ultrasound imaging system arranged in accordance with principles of the present disclosure.
Fig. 2 is a block diagram illustrating a workflow for predicting motion and performing stabilized zooming in an ultrasound image, according to principles of the present disclosure.
Fig. 3 is a flow diagram illustrating a process for modifying a zoom region of interest, according to principles of the present disclosure.

### DETAILED DESCRIPTION OF EMBODIMENTS

The following description of certain examples is merely illustrative in nature and is in no way intended to limit the invention or its applications or uses. In the following detailed description of examples of the present apparatuses, systems, and methods, reference is made to the accompanying drawings which form a part hereof, and in which are shown by way of illustration specific examples in which the described apparatuses, systems and methods may be practiced. These examples are described in sufficient detail to enable those skilled in the art to practice the presently disclosed apparatuses, systems and methods, and it is to be understood that other examples may be utilized and that structural and logical changes may be made without departing from the spirit and scope of the present disclosure. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present technology is defined only by the appended claims.

Stable imaging of targets can be challenging due to patient, operator, and/or physiological motion. Clinically, poor imaging stability can lead to the loss of targets from the field of view (FOV), resulting in non-diagnostic acquisitions and/or disruptions in interventional or procedural guidance. This is especially problematic in zoomed imaging modes, where the FOV is smaller and the perceived motion of targets is amplified with magnification.

The present disclosure describes systems and related methods for motion prediction for stabilized zoom in medical images. For example, the disclosed technology predicts motion of target object, such as a target anatomy or a medical treatment device, which can be caused by deformation and/or displacement of tissue or movement of an imaging device (e.g., a US probe), and the disclosed technology uses the predicted motion to stabilize the target object in medical images. As used herein, stabilizing the target object can refer to centering and/or otherwise maintaining a position of the target object in a displayed medical image. In other words, the target object can appear to remain stationary in the displayed medical image, while surrounding tissue and/or objects appear to move relative to the target object. The disclosed technology can be applied, for example, in B-mode imaging for tracking various anatomical landmarks and/or devices. Additionally or alternatively, the disclosed technology can be applied to other imaging modes, such as color Doppler for tracking cardiac jets and/or small vessels. The disclosed technology can be applied using two-dimensional (2D) or three-dimensional (3D) imaging. Although examples are described herein with reference to US imaging, other imaging modalities can also be used.

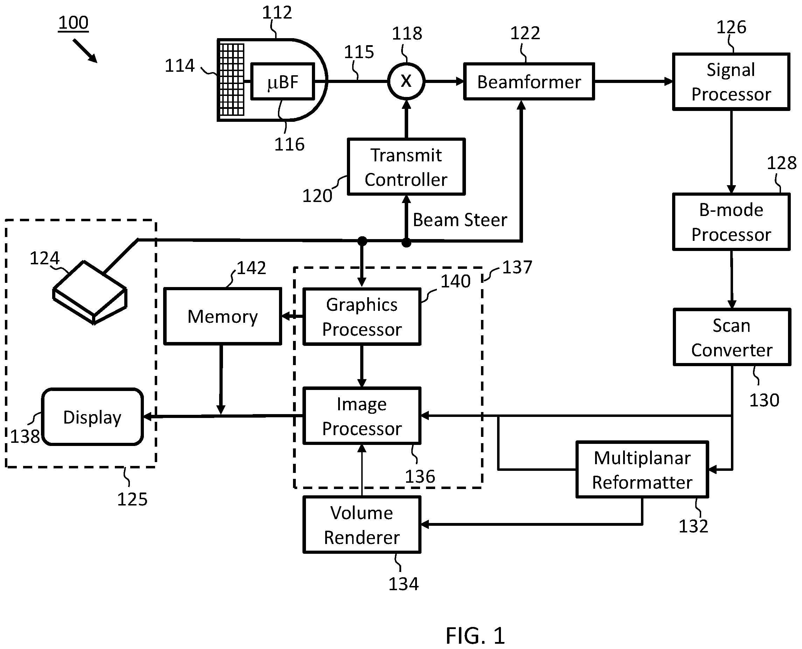

Fig. 1 is a block diagram of an ultrasound imaging system 100 arranged in accordance with principles of the present disclosure. In the ultrasound imaging system 100 of Fig. 1, an ultrasound probe 112 includes a transducer array 114 for transmitting ultrasonic waves and receiving echo information. The transducer array 114 may be implemented as a linear array, convex array, a phased array, and/or a combination thereof. The transducer array 114, for example, can include a two-dimensional array (as shown) of transducer elements capable of scanning in both elevation and azimuth dimensions for 2D and/or 3D imaging. The transducer array 114 may be coupled to a microbeamformer 116 in the probe 112, which controls transmission and reception of signals by the transducer elements in the array. In this example, the microbeamformer 116 is coupled by the probe cable to a transmit/receive (T/R) switch 118, which switches between transmission and reception and protects the main beamformer 122 from high-energy transmit signals. In some embodiments, the T/R switch 118 and other elements in the system can be included in the ultrasound probe 112 rather than in a separate ultrasound system base. In some embodiments, the ultrasound probe 112 may be coupled to the ultrasound imaging system via a wireless connection (e.g., WiFi, Bluetooth).

The transmission of ultrasonic beams from the transducer array 114 under control of the microbeamformer 116 is directed by the transmit controller 120 coupled to the T/R switch 118 and the beamformer 122, which receives input from the user's operation of the user interface (e.g., control panel, touch screen, console) 125. The user interface 125 may include soft and/or hard controls. One of the functions controlled by the transmit controller 120 is the direction in which beams are steered. Beams may be steered straight ahead from (orthogonal to) the transducer array 114, or at different angles for a wider field of view. The partially beamformed signals produced by the microbeamformer 116 are coupled via channels 115 to a main beamformer 122 where partially beamformed signals from individual patches of transducer elements are combined into a fully beamformed signal. In some embodiments, microbeamformer 116 is omitted and the transducer array 114 is coupled via channels 115 to the beamformer 122. In some embodiments, the system 100 may be configured (e.g., include a sufficient number of channels 115 and have a transmit/receive controller programmed to drive the transducer array 114) to acquire ultrasound data responsive to a plane wave or diverging beams of ultrasound transmitted toward the subject. In some embodiments, the number of channels 115 from the ultrasound probe may be less than the number of transducer elements of the transducer array 114 and the system may be operable to acquire ultrasound data packaged into a smaller number of channels than the number of transducer elements.

The beamformed signals are coupled to a signal processor 126. The signal processor 126 can process the received echo signals in various ways, such as bandpass filtering, decimation, I and Q component separation, and harmonic signal separation. The signal processor 126 may also perform additional signal enhancement such as speckle reduction, signal compounding, and noise elimination. The processed signals are coupled to a B-mode processor 128, which can employ amplitude detection for the imaging of structures in the body. The signals produced by the B-mode processor 128 are coupled to a scan converter 130 and a multiplanar reformatter 132. The scan converter 130 arranges the echo signals in the spatial relationship from which they were received in a desired image format. For instance, the scan converter 130 may arrange the echo signal into a two-dimensional (2D) sector-shaped format, or a pyramidal three-dimensional (3D) image. The multiplanar reformatter 132 can convert echoes, which are received from points in a common plane in a volumetric region of the body into an ultrasonic image of that plane, as described in U.S. Pat. No. 6,443,896 (Detmer).

A volume renderer 134 converts the echo signals of a 3D data set into a projected 3D image as viewed from a given reference point, e.g., as described in U.S. Pat. No. 6,530,885 (Entrekin et al.) The 2D or 3D images may be coupled from the scan converter 130, multiplanar reformatter 132, and volume renderer 134 to at least one processor 137 for further image processing operations. For example, the at least one processor 137 may include an image processor 136 configured to perform further enhancement and/or buffering and temporary storage of image data for display on an image display 138. The display 138 may include a display device implemented using a variety of display technologies, such as LCD, LED, OLED, or plasma display technology. The at least one processor 137 may include a graphics processor 140, which can generate graphic overlays for display with the ultrasound images. These graphic overlays can contain, e.g., standard identifying information such as patient name, date and time of the image, imaging parameters, and the like. For these purposes the graphics processor 140 receives input from the user interface 125, such as a typed patient name. The user interface 125 can also be coupled to the multiplanar reformatter 132 for selection and control of a display of multiple multiplanar reformatted (MPR) images. The user interface 125 may include one or more mechanical controls, such as buttons, dials, a trackball, a physical keyboard, and others, which may also be referred to herein as hard controls. Alternatively or additionally, the user interface 125 may include one or more soft controls, such as buttons, menus, soft keyboard, and other user interface control elements implemented for example using touch-sensitive technology (e.g., resistive, capacitive, or optical touch screens). One or more of the user controls may be co-located on a control panel 124. For example one or more of the mechanical controls may be provided on a console and/or one or more soft controls may be co-located on a touch screen, which may be attached to or integral with the console. The display 138 and the user interface 125 can be included in an I/O component, via which outputs are provided by the system 100 and/or inputs are received by the system 100

The at least one processor 137 (e.g., the image processor 136, the graphics processor 140, or a different processor) may also perform functions associated with predicting motion for stabilized zoom, as described herein. For example, the at least one processor 137 can identify a zoom region of interest and a target object in the zoom region of interest. The at least one processor 137 can cause a set of medical images to be stored and/or streamed to a memory 142, which can include a first-in first-out buffer, and the at least one processor 137 can determine movement of the target object in the set of medical images. Based on the movement of the target object, the at least one processor 137 predicts a location of the target object motion in a subsequent image and modifies the target region of interest to stabilize the target object (e.g., in a displayed image on the display 138).

Although described as separate processors, it will be understood that the functionality of any of the processors described herein may be implemented in a single processor (e.g., a CPU or GPU implementing the functionality of processor 137) or fewer number of processors than described in this example. In some embodiments, the at least one processor 137 may be hardware-based (e.g., include multiple layers of interconnected nodes implemented in hardware). In some embodiments, the at least one processor 137 may be implemented at other processing stages, e.g., prior to the processing performed by the image processor 136, volume renderer 134, multiplanar reformatter 132, and/or scan converter 130. In some embodiments, the at least one processor 137 may be implemented to process ultrasound data in the channel domain, beamspace domain (e.g., before or after beamformer 122), the IQ domain (e.g., before, after, or in conjunction with signal processor 126), and/or the k-space domain. As described, in some embodiments, functionality of two or more of the processing components (e.g., beamformer 122, signal processor 126, B-mode processor 128, scan converter 130, multiplanar reformatter 132, volume renderer 134, at least one processor 137, image processor 136, graphics processor 140, etc.) may be combined into a single processing unit or divided between multiple processing units. The processing units may be implemented in software, hardware, or a combination thereof. For example, the at least one processor 137 may include one or more graphical processing units (GPU). In another example, beamformer 122 may include an application specific integrated circuit (ASIC).

The at least one processor 137 can be coupled to one or more computer-readable media (e.g., memory 142) included in the system 100, which can be non-transitory. The one or more computer-readable media can carry instructions and/or a computer program that, when executed, cause the at least one processor 137 to perform operations described herein. A computer program may be stored/distributed on any suitable non-transitory medium, such as an optical storage medium or a solid-state medium supplied together with or as part of other hardware, but may also be distributed in other forms, such as via the Internet or other wired or wireless telecommunication systems. Furthermore, the different embodiments can take the form of a computer program product accessible from a computer-readable medium providing program code for use by or in connection with a computer or any device or system that executes instructions. For the purposes of this disclosure, a computer-readable medium can generally be any tangible apparatus that can contain, store, communicate, propagate, or transport the program for use by or in connection with the instruction execution device. The computer-readable medium can be, for example, without limitation, an electronic, magnetic, optical, electromagnetic, infrared, or semiconductor system, or a propagation medium. Non-limiting examples of a computer readable medium include a semiconductor or solid state memory, magnetic tape, a removable computer diskette, a random access memory (RAM), a read-only memory (ROM), a rigid magnetic disk, and/or an optical disk. Optical disks may include compact disk read only memory (CD-ROM), compact disk-read/write (CD-R/W), and/or DVD.

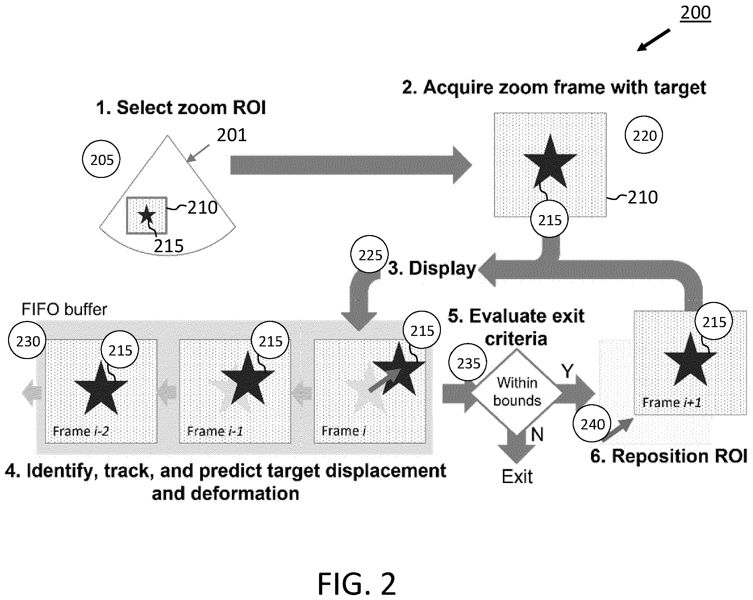

Fig. 2 is a block diagram illustrating a workflow 200 for predicting motion and performing stabilized zooming in an ultrasound image, according to principles of the present disclosure. The workflow 200 can be performed using the system 100 of Fig. 1.

The workflow 200 begins at operation 205, where an image 201 is acquired, for example by an ultrasound probe, such as probe 112, and a zoom ROI 210 is selected. The zoom ROI 210 can be a portion of the acquired image 201 that includes a target object 215, such as a target anatomy or a medical treatment device (e.g., a needle or catheter). The selection of the zoom ROI 210 can be performed in various ways, such as by receiving a selection from a user via a user interface (e.g., user interface 125) or automatically selecting the zoom ROI 210 based on identifying the target object 215 in the acquired image 201.

The workflow 200 proceeds to operation 220, where a frame is acquired, the frame comprising the zoom ROI 210 with the object 215. The frame acquired at operation 220 can be a zoomed-in view of the zoom ROI 210 and the object 215. The zoomed-in view can be acquired by cropping the image 201 acquired at operation 205 and/or re-acquiring a portion of image 201 at a location corresponding to the zoom ROI 210 at operation 205. Additionally or alternatively, a non-uniform acquisition may be performed where low-resolution imaging is used to acquire a portion of the image 201 outside the zoom ROI 210 and a portion of the image 201 inside the zoom ROI 210 is acquired using high-resolution imaging. In some embodiments, low-resolution imaging may be achieved by broad beam transmits, decreased line density, and/or other settings. In some embodiments, high-resolution imaging may be achieved by focused transmits, increased line density, and/or other settings. The workflow 200 proceeds to operation 225, where the frame acquired at operation 220 is displayed (e.g., on display 138. The workflow 200 proceeds to operation 230, where a plurality of frames, Frame *i* through Frame *i-n,* are acquired and adaptively stored in a first-in first-out (FIFO) buffer, which may be included in a memory, such as memory 142. In some embodiments, the frames may include raw imaging data such as radiofrequency data, IQ data, or the like. Although three frames are illustrated in the operation 240, any number of frames can be acquired and stored. Each frame of Frame i through Frame *i-n* includes the target object 215. In some instances, the target object 215 may appear to move in successive frames, such as due to movement or deformation of the target object 215, movement or deformation of surrounding tissue, and/or movement of an imaging device (e.g., a US probe used to acquire the frames). The plurality of frames of the FIFO buffer are used to identify and track the target object 215 and to predict motion of the object 215 based on a position of the object 215 in successive frames of the plurality of frames. In some implementations, the first-in first-out buffer can be configured to store up to a preset number of frames (e.g., 10 frames, 100 frames, 1000 frames).

The workflow 200 proceeds to operation 235, where exit criteria for the workflow 200 are evaluated. For example, an exit criterion may include whether the target object 215 remains within bounds and/or within a threshold area or distance, such as within an area included in the image 201 acquired at operation 205, whether or not the object 215 is present in a current frame (e.g., within a current zoom region of interest 210). If it is determined at operation 235 that the target object 215 is not within bounds (or if an exit criterion is otherwise detected) then the workflow 200 may be terminated. For example, the disclosed system may cease tracking of the target object 215 and/or display a notification to a user, such as a notification that the target object 215 is out of bounds. If, at operation 235, no exit criterion is identified, then the workflow 200 proceeds to operation 240.

At operation 240, a new Frame i+1 is acquired using a repositioned and/or modified zoom ROI, which is repositioned based on the motion of the target object 215 predicted at operation 230. For example, the repositioned zoom ROI at operation 240 can re-center the target object 215, such that the object 215 appears to remain stationary in the center of the displayed image while surrounding tissue appears to move relative to the target object 215. In some implementations, the new Frame i+1 can be acquired without repositioning an imaging device, such as a US probe. The workflow 200 returns to operation 225, where the new Frame i+1 is displayed.

The workflow 200 can be repeated any number of times, such that the object 215 can be imaged in successive repositioned ROIs 210 to appear to remain stationary while maintaining a zoomed-in view.

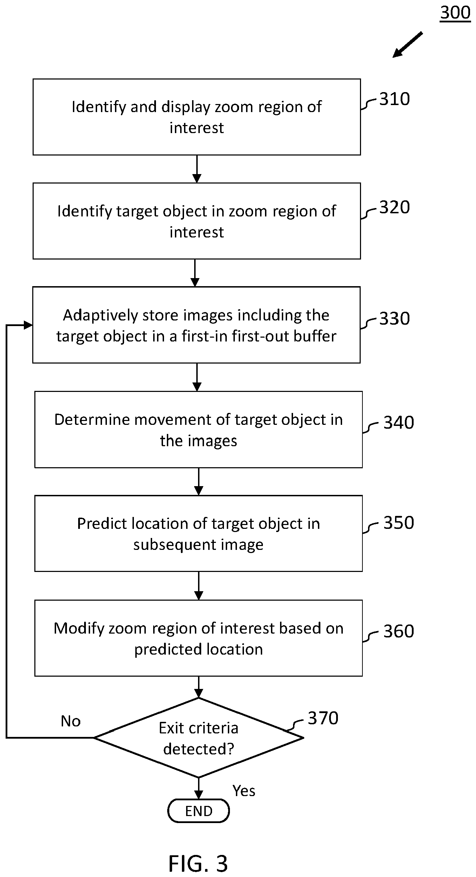

Fig. 3 is a flow diagram illustrating a process 300 for modifying a zoom region of interest, according to principles of the present disclosure. The process 300 can be performed, for example, using the at least one processor 137 of the system 100 of Fig. 1. For example, the process 300 can be used to stabilize a target object on the display 138 within a zoom region of interest in images acquired using the ultrasound probe 112 of Fig. 1. In some embodiments, the process 300 may be included in workflow 200.

The process 300 begins at block 310, where a zoom region of interest within a medical image is identified and displayed. For example, an ultrasound image can be displayed, and a zoomed-in view of the ultrasound image can be selected by a user or automatically, such as to magnify a region of interest where a target object is present. Displaying the zoom region of interest can include displayed a magnified view of a larger image or acquiring a sub-image of the larger image.

The process 300 proceeds to block 320, where a target object is identified in the zoom region of interest. The target object can be identified automatically and/or selected by a user. Identifying the target object can include performing feature localization. The target object can be, for example, a target anatomy and/or a medical treatment device, such as a needle or catheter. The target object can be identified in various ways, such as masking or soft segmentation using thresholding with threshold values based on expected imaging properties of the target object (e.g., signal intensity, signal-to-noise ratio, flow velocity, flow power, backscatter-derived properties). In some embodiments, classification algorithms based on artificial intelligence, computer vision, or classical image processing techniques may be used to identify the target object. In some implementations, identifying the target object can include generating a mask. In other embodiments, identifying the target object may be omitted and tracking may be performed across an entire zoom ROI.

The process 300 proceeds to block 330, where a set of images is adaptively stored in a first-in first-out buffer. For example, a plurality of images can be acquired that include the zoom region of interest and/or the target object, and the acquired images can be stored in the first-in first-out buffer based on the order in which they are acquired. In some implementations, membership in the set of images can be changed, such as by storing up to a preset number of images (e.g., 10 images, 100 images, a 1000 images) and discarding images beyond the preset number of images on a first-in first-out basis.

The process 300 proceeds to block 340, where movement of the target object is determined in the set of images. For example, the target object can be identified in each of the images in the set of images (e.g., using feature localization), and a position of the target object can be determined over time. The movement of the target object within the images in the set of images can correspond to displacement or deformation of the target object, displacement or deformation of surrounding tissue, and/or motion of an imaging device, such as an ultrasound probe. The movement of the target object can be determined in various ways, such as using displacement tracking via motion estimation methods (e.g., block matching, phase correlation, optical flow). In some implementations, movement of the target object can be determined using artificial intelligence and/or computer vision techniques. In some implementations, movement of the target object can be determined using displacement and/or deformation tracking via non-rigid or rigid image and/or mesh registration. Movement of the target object can be determined using estimated displacements and/or deformations, which can be applied in subsequent steps either directly or after conditioning (e.g., using outlier detection and omission, interpolation between outliers, spatial and/or temporal averaging, curve fitting). Determining movement of the target object can use various tracking dimensions, including elevational, axial and/or lateral dimensions. In some implementations, determining movement of the target object can include applying a mask generated at block 320 to determine or estimate movement (e.g., deformation or displacement) of the target object.

The process 300 proceeds to block 350, where a location of the target object in a subsequent image is predicted based on the movement determined at block 340. For example, a rate and/or direction of movement can be determined, and the rate and/or direction can be used to extrapolate a location of the target object in a next image acquired after the images in the set of images and/or additional subsequent images.

The process 300 proceeds to block 360, where the zoom region of interest identified at block 310 is modified based on the location predicted at block 350 to stabilize the target object. Modifying the zoom region of interest may include modifying a location of the zoom region of interest and/or dimensions of the zoom region of interest. For example, the modified zoom region of interest can maintain the target object in substantially the same location on a display, while tissue and/or objects surrounding the target object appear to move relative to the displayed target object. The modified zoom region of interest can be displayed, such as by displaying at least a portion of the subsequent image and/or acquiring a sub-image of the subsequent image. In some implementations, displaying the modified zoom region of interest can include displaying at least a portion of a previously-acquired image (e.g., retroactively displaying the modified zoom region of interest). In some implementations, modifying the zoom region of interest can include determining a degree of zooming to stabilize the target object (e.g., to zoom in or zoom out).

The process 300 proceeds to decision block 370, where it is determined whether one or more exit criteria are detected. If the one or more exit criteria are detected, then the process 300 terminates. If the one or more exit criteria are not detected, then the process 300 proceeds to block 300 to perform an additional iteration of the adaptive storing of the set of images. The adaptive storing of the set of images, the determining the movement of the target object, the predicting of the location of the target object in the subsequent image, and the modifying of the zoom region of interest can be performed iteratively until the one or more exit critiera are detected at block 370. An exit criterion can include, for example, movement of the target object beyond a threshold distance and/or movement of the target object outside of a detectable region (e.g., outside of a region being imaged). Exit criteria can be based on one or more confidence metrics, such as a confidence metric derived from identifying the target object (e.g., whether the target object can be identified beyond a threshold confidence metric) and/or a confidence metric based on one or more tracking variables (e.g., correlation coefficient, mean squared error). In some implementations, after the iterative operations are terminated, the iterative operations may be resumed if one or more resumption criteria are detected (e.g., if the target object is detected again). Other exit criteria can include various user and/or system inputs, such as repositioning a region of interest, pressing a button, exceeding a time threshold, and so forth.

In some implementations, the process 300 can include applying non-uniform acquisition to achieve higher resolution imaging (e.g., focused transmits, increased line density) inside the zoom region of interest and lower resolution padding (e.g., broad beam transmits, decreased line density) for target object tracking outside the zoom region of interest.

In some implementations, operations of the process 300 may use various ultrasound data, such as RF signals and/or non-visible ultrasound data, such as for identifying the zoom region of interest, identifying the target object, determining the movement of the target object and/or predicting the location of the target object.

The process 300 can be performed in any order, including performing one or more operations in parallel and/or repeating one or more operations. Additionally, operations can be added to or removed from the process 300 without deviating from the teachings of the present disclosure.

The systems and related methods disclosed herein may provide for motion prediction for stabilized zoom in medical images. This may improve a user's ability to view a target within a ROI and/or reduce instances of "losing" a desired target during an imaging exam. While the examples provided herein refer to zoom ROI, the principles of the present disclosure may be applied to other ROI. For example, a ROI may be selected from a B-mode image where Doppler images are acquired, and the location of a target may be maintained within the Doppler ROI.

In various examples where components, systems and/or methods are implemented using a programmable device, such as a computer-based system or programmable logic, it should be appreciated that the above-described systems and methods can be implemented using any of various known or later developed programming languages, such as "Python", "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, examples or processes described herein may be combined with one or more other examples, examples and/or processes or be separated and/or performed amongst separate devices or device portions in accordance with the present systems, devices and methods.

Finally, the above-discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended spirit and scope of the present systems and methods as set forth in the claims that follow. Accordingly, the specification and drawings are to be regarded in an illustrative manner and are not intended to limit the scope of the appended claims.

## Claims

1. An ultrasound imaging system comprising:
an ultrasound probe configured to acquire ultrasound image data; and
at least one processor in communication with the ultrasound probe, the at least one processor configured to:
identify a zoom region of interest in an image of the ultrasound image data;
cause display of the zoom region of interest in the image;
identify a target object in the zoom region of interest;
cause a set of images of the ultrasound image data to be stored in a first-in first-out buffer, wherein each image in the set of images includes the target object;
determine movement of the target object in the set of images;
predict a location of the target object in a subsequent image based on the determined movement of the target object in the set of images;
modify the zoom region of interest based on the predicted location of the target object in the subsequent image; and
cause display of the modified zoom region of interest in the subsequent image.

2. The ultrasound imaging system of claim 1, wherein the zoom region of interest is modified based on the predicted location of the target object in the subsequent image such that the target object is displayed on a display in a same location in the image and the subsequent image.

3. The ultrasound imaging system of claim 1 or 2, wherein the movement of the target object corresponds to at least one of displacement of the target object, deformation of the target object, displacement of tissue, deformation of tissue, or motion of the ultrasound probe between images of the set of images.

4. The ultrasound imaging system of any of claims 1 to 3, wherein the target object and/or the zoom region of interest is identified automatically.

5. The ultrasound imaging system of any of claims 1 to 4, wherein the target object is identified based on at least one of backscatter intensity, signal-to-noise ratio, flow velocity, or flow power.

6. The ultrasound imaging system of any of claims 1 to 5, wherein the target object comprises a target anatomy or a medical treatment device.

7. The ultrasound imaging system of any of claims 1 to 6, wherein storing the set of images includes changing membership in the set of images.

8. The ultrasound imaging system of any of claims 1 to 7, wherein the at least one processor is configured to iteratively perform the storing of the set of images, the determining the movement of the target object, the predicting of the location of the target object in the subsequent image, and the modifying of the zoom region of interest.

9. The ultrasound imaging system of claim 8, wherein the at least one processor is configured to terminate the iterative performing in response to detecting an exit criterion, wherein preferably the exit criterion comprises movement of the target object beyond a threshold, receiving a user input, or receiving a system input.

10. The ultrasound imaging system of claim 9, wherein detecting the exit criterion comprises calculating a confidence metric based on at least one tracking variable, and wherein the tracking variable includes at least one of a correlation coefficient or a mean squared error.

11. The ultrasound imaging system of any of claims 1 to 10, wherein the at least one processor is configured to cause at least one of non-uniform transmit sequencing or non-uniform receive beamforming to acquire the ultrasound image data at a first resolution within the zoom region of interest and at a second resolution outside of the zoom region of interest, wherein the first resolution is higher than the second resolution.

12. The ultrasound imaging system of any of claims 1 to 11, wherein causing display of the zoom region of interest comprises magnifying the image or acquiring a different image, and/or wherein causing display of the modified zoom region of interest in the subsequent image comprises presenting at least a portion of a previously-acquired image.

13. The ultrasound imaging system of any of claims 1 to 12, wherein modifying the zoom region of interest based on the predicted location of the target object in the subsequent image comprises determining a degree of zooming in the subsequent image.

14. A computer-implemented method of modifying a zoom region of interest in medical images, the method comprising:
identifying a zoom region of interest in an image of medical imaging data;
causing display of the zoom region of interest in the image;
identifying a target object in the zoom region of interest;
adaptively storing a set of images of the medical imaging data in a first-in first-out buffer, wherein each image in the set of images includes the target object;
determining movement of the target object in the set of images;
predicting a location of the target object in a subsequent image based on the determined movement of the target object in the set of images;
modifying the zoom region of interest based on the predicted location of the target object in the subsequent image; and
causing display of the modified zoom region of interest in the subsequent image.

15. A non-transitory computer-readable medium carrying instructions that, when executed, cause a processor to perform operations to perform the computer-implemented method of claim 14.
